(19) **Europäisches Patentamt / European Patent Office / Office européen des brevets**

(11) **EP 1 694 384 B1**

(12) **EUROPEAN PATENT SPECIFICATION**

(45) Date of publication and mention
of the grant of the patent:
**18.02.2009 Bulletin 2009/08**

(51) Int Cl.:
***A61M 5/145*** (2006.01)     ***F16H 19/02*** (2006.01)

(21) Application number: **04805986.9**

(22) Date of filing: **09.12.2004**

(86) International application number:
**PCT/GB2004/005166**

(87) International publication number:
**WO 2005/056086 (23.06.2005 Gazette 2005/25)**

(54) **AN IMPROVED FEED MECHANISM FOR A MEDICAL DEVICE**

VERBESSERTER ZUFUHRMECHANISMUS FÜR EIN MEDIZINPRODUKT

MECANISME D'ALIMENTATION AMELIORE POUR UN DISPOSITIF MEDICAL

(84) Designated Contracting States:
**AT BE BG CH CY CZ DE DK EE ES FI FR GB GR
HU IE IS IT LI LT LU MC NL PL PT RO SE SI SK TR**

(30) Priority: **09.12.2003 GB 0328558**

(43) Date of publication of application:
**30.08.2006 Bulletin 2006/35**

(73) Proprietor: **Zimed Holdings Limited
4 More London Riverside
London SE1 2AU (GB)**

(72) Inventor: **GALLAGHER, George,
Zi Medical Plc
St Asaph,
Denbighshire LL17 0LJ (GB)**

(74) Representative: **Lees, Kate Jane et al
Marks & Clerk
Tower Building
Water Street
Liverpool L3 1BA (GB)**

(56) References cited:
**FR-A- 1 562 416         FR-A- 2 541 395
JP-A- 10 297 478         US-A- 4 253 342
US-A- 5 006 112**

**Description**

The present invention relates to an improved feed mechanism for a medical device, particularly but not exclusively a syringe driver or pump.

Syringe drivers or plumps are well known. They are small, lightweight, battery operated machines that are designed to administer subcutaneous infusions of a prescribed amount of medication over a given period. A syringe driver basically consists of the machine itself, a syringe containing the medicine to be administered which is attached to the machine and a thin piece of tubing attached to the syringe which has a needle at the end of it. Syringe drivers are often provided with both the machine and the syringe contained within a housing to increase the portability of the device.

The drive mechanism for driving the plunger through the syringe barrel to dispense medication generally consists of a motor, gears and a threaded shaft. The motor causes rotation of the threaded shaft which, via an actuator attached thereto, effects movement of the plunger (for example, see US 5,006,112). Once the required medication has been dispensed, it is necessary to manually reset the syringe driver by pulling back the actuator and syringe plunger to the required degree. Conventionally, this is achieved by the provision of two half nuts around the threaded shaft, the manual disengagement of which enables the actuator to be moved back to the end of the shaft to allow the plunger to be reset. However, these nuts are subject to a large amount of wear and tear and thus require frequent replacement. Furthermore, once the nuts have become worn, the shaft will still rotate placing a load on the motor but without imparting any movement to the actuator.

It is an object of the present invention to provide an improved feed mechanism for a medical device, particularly but not exclusively a syringe driver or pump that aims to overcome, or at least alleviate the abovementioned drawbacks.

Accordingly, a first aspect of the present invention provides a syringe driver assembly comprising driver means for imparting translational movement to a syringe plunger, the driver means comprising a motor driven unthreaded shaft, at least one bearing mounted obliquely to the shaft and having at least one point of contact therewith, and an actuator linked to the at least one bearing for contacting a thumb plate of the plunger wherein rotation of the shaft causes movement of the at least one bearing along the shaft to affect movement of the actuator.

If only a single bearing is provided, the shaft should be supported at one or more points along its length by a rotary member. The rotary member should be provided on the opposite side of the shaft to the contact point of the bearing.

However, in a preferred embodiment, more than one bearing is mounted obliquely to the shaft, especially three bearings, each having a bore through which the shaft extends. More preferably, alternate bearings are set at the same angle relative to the shaft and adjacent bearings are set at an opposing angle relative to the shaft. The bore of each bearing should be larger than the outer circumference of the shaft to result in each bearing being oversized with respect to the shaft, thereby creating clearance between the shaft and each bearing.

The inner profile of the bearings may be flat or pointed. Preferably, bearings having a flat inner profile with a chamfered inner race are used, each bearing being angled with respect to the shaft such that it contacts the shaft at at least two points. In the case of three bearings being used, preferably the central bearing contacts the opposing side of the shaft to the outer bearings and is at an opposite angle thereto. More preferably still, the outer bearings contact the bottom of the shaft and the central bearing contacts the top of the shaft or vice versa.

The bearings are preferably made of a hardened steel, such as stainless steel. More preferably, the angle of inclination of each bearing relative to the shaft is less than 45 degrees, preferably less than 20 degrees, more preferably less than 15 degrees, especially less than 5 degrees.

In alternative embodiment, the inclined bearings may be symmetrically spaced in one plane perpendicular to the shaft axis, the outer races of the bearings making radial contact with the shaft. In this embodiment, each bearing is preferably sprung loaded with respect to the shaft.

It is preferable for the bearings to be housed within a carriage that is moveable with respect to the shaft. Each bearing may be housed within its own casing that surrounds the shaft, the casings being interconnectable to form the carriage. Preferably, the carriage is connected to the actuator that contacts the member, such as the thumbplate or plunger head of the syringe. The carriage is preferably provided with guides. The carriage and actuator may form an integral component.

It is preferable for the assembly to provide an axial force of at least 10 Newtons, more preferably at least 15 Newtons. In fact, the assembly has been found to provide a force in excess of 50 Newtons.

The assembly may be provided with means for manually disengaging one or more of the bearings to enable the bearings and/or carriage to slide with respect to the shaft. The middle bearing may be spring loaded with respect to the shaft whereby operation of the spring mechanism disengages the middle bearing from the shaft or manual disengagement may be caused by movement of a casing that carries one or more of the bearings in a direction transverse to the shaft, for example by means of a cam and lever. The carriage or actuator may be provided with a means for operation of the spring or cam.

Alternatively, the assembly may be provided with automatic means for reversing the direction of travel of the carriage and actuator. For example, means may be provided to reverse the angle of inclination of the bearings with respect to the shaft to cause the bearings to travel in the opposite direction without changing the direction of rotation of the shaft.

Preferably, means is provided to detect any backward pressure exerted on the syringe driver assembly. More preferably, detection of a backward pressure above a certain threshold results in stopping of the motor.

Additionally, means may be provided for allowing calibration of the system. Preferably, at least one of the bearings is provided with adjustable biasing means. For example, a grub screw and compression plate may be provided in communication with one of the bearings which can be used to increase or decrease the load of the bearing on the shaft.

For a better understanding of the present invention and to show more clearly how it may be carried into effect, reference will now be made by way of example only to the accompanying drawings in which:-

Figure 1 is a schematic perspective view of the internal components of a syringe driver assembly according to one embodiment of the present invention;

Figure 2 is a perspective front view of a carriage and drive shaft for a syringe driver feed mechanism according to one embodiment of the present invention;

Figure 3 is perspective view of the housing and drive shaft of Figure 2 with one half of the housing removed to show the internal components thereof;

Figure 4 is a plan view of the housing and drive shaft of Figure 2 with half of the housing removed to show the internal components thereof;

Figure 5 is a cross-sectional view through the carriage of Figure 2;

Figure 6 is a schematic diagram illustrating the mounting of the bearings relative to the driver shaft;

Figure 7 is an exploded side perspective view of a feed mechanism according to a second embodiment of the present invention; and

Figure 8 is an unexploded end perspective view of the feed mechanism shown in Figure 7.

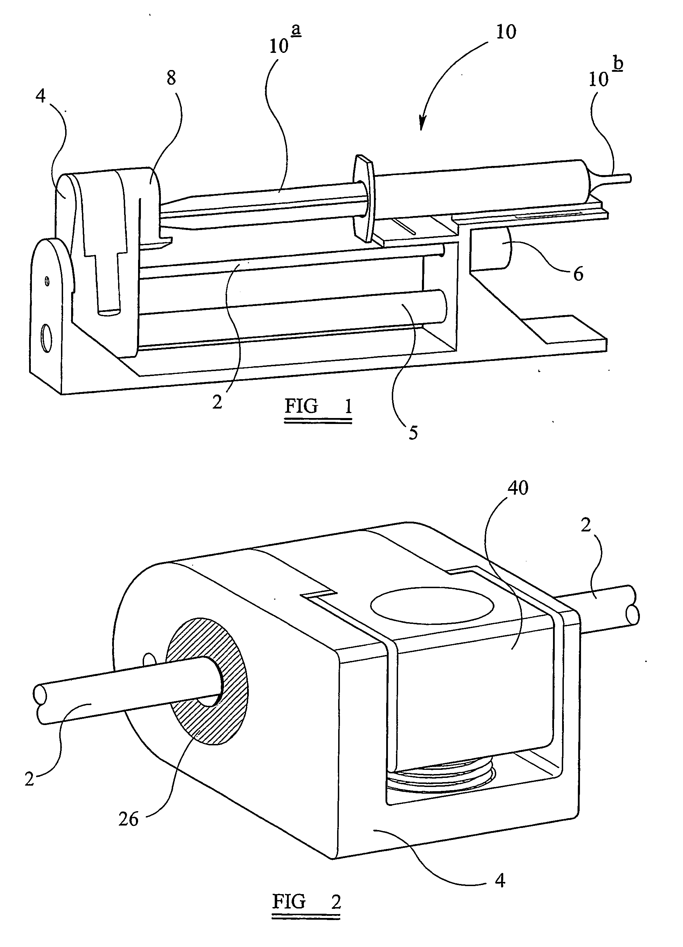

Referring to Figure 1 of the accompanying drawings, the internal components of a syringe driver are shown comprising a drive shaft 2 carrying a carriage 4 mounted on guides 5, the shaft being driven by a motor 6. It is to be appreciated that the components would be contained within a housing but this has been omitted from the drawings for the sake of simplicity. The carriage 4 is connected to an actuator 8 that abuts the plunger 10a of a syringe 10 when one is placed within the syringe driver assembly. Movement of the actuator causes displacement of the plunger thereby causing fluid to be dispensed from the nozzle 10b of the syringe, as required.

Conventionally, a threaded shaft is used to convert the rotary motion of the motor driven shaft into linear motion. However, in order to reset the shaft with the actuator in the desired position to dispense fluid from a syringe, two half-nuts are provided around the shaft that may be disengaged therefrom in order for the drive carriage to be moved back to its desired position. This is undesirable because the nuts are subject to a large amount of wear and tear, necessitating frequent replacement of the parts. Furthermore, the shaft will continue to rotate when the nuts are worn and no longer grip the shaft causing a load to be placed on the motor without imparting any movement to the syringe plunger. This also results in the syringe driver appearing as if it is administering fluid from the syringe when in fact it is not. The present invention provides an alternative feed mechanism for driving the plunger of the syringe that aims to overcome the abovementioned drawbacks.

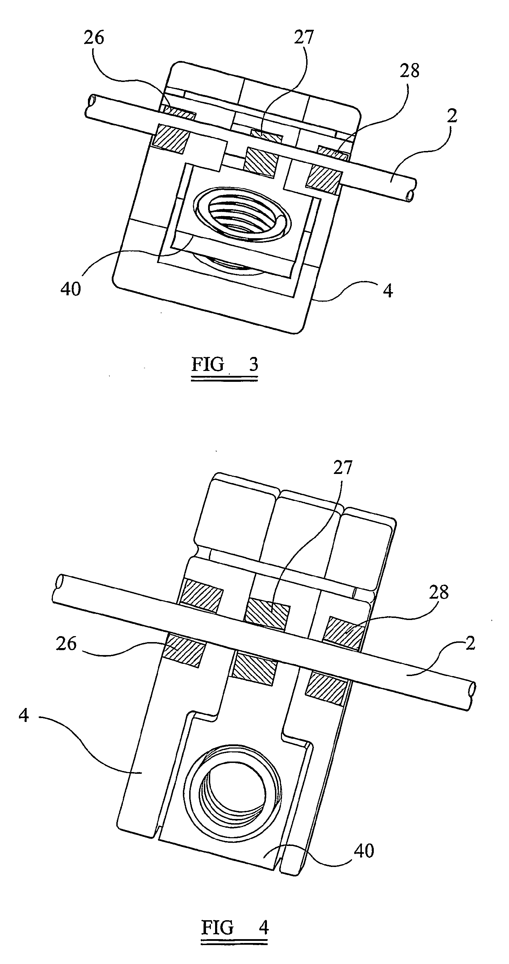

Referring to Figures 1 to 5 of the accompanying drawings, the components of a feed mechanism for driving a syringe according to one embodiment of the present invention is illustrated. The syringe driver comprises a smooth, unthreaded shaft 2 or bar that is rotated by means of a motor 6. The shaft runs through a carriage 4 that is mounted on guides 5, the carriage containing three anti-friction bearings 26, 27, 28 that are offset with respect to the shaft. An actuator 8 is connected to the carriage 4 whereby movement of the carriage imparts movement to the actuator. The actuator abuts the syringe plunger of a syringe to affect movement thereof to result in fluid being dispensed from the syringe.

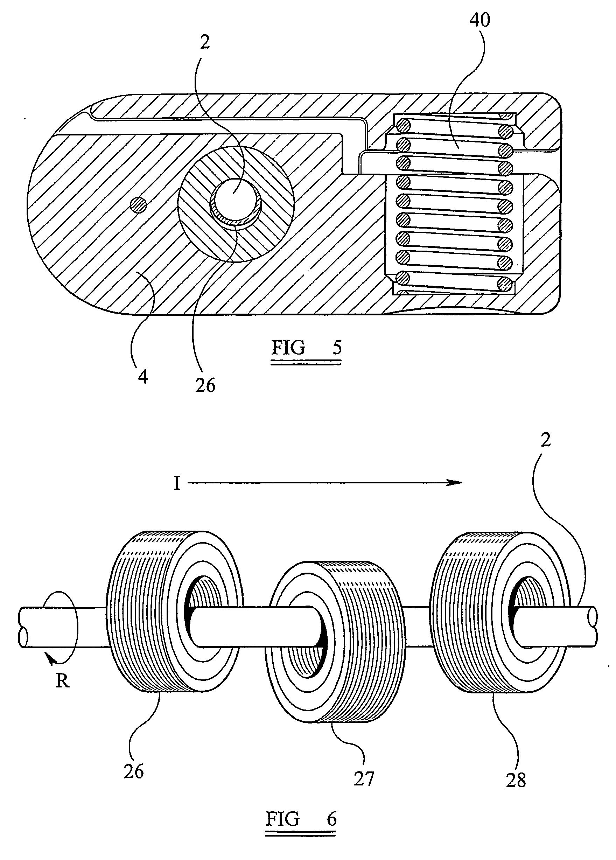

The rotary motion of the shaft is converted into linear motion by means of the three bearings 26, 27 and 28 that are fixed within the carriage. The three bearings are configured such as to provide clearance between each bearing and the shaft. The two outer bearings 26, 28 are fixed at the same angle relative to the longitudinal axis of the shaft and the middle bearing 27 is fixed at an equal and opposite angle with respect to the shaft, resulting in the outer bearings running on their corresponding edges with respect to the shaft with the middle bearing running on its opposing edge. This causes the bearings to "roll" along the length of the shaft thereby converting the rotary input R provided by the motor-driven shaft into a linear output T (see Figure 6). Thus, this arrangement enables the controlled feed of the carriage, and thus the actuator, in one direction only, by means of the rotary motion of the shaft. The actuator engages with the plunger of the syringe thereby enabling controlled discharge of fluid within the syringe.

The forces on the shaft are collectively externally balanced and the shaft is supported in a bearing system that fixes it on its axial and radial axes but allows low friction rotation. A stepping motor is preferably used to drive rotation of the shaft. The shaft and the bore of the bearings should be of hardened steel, such as stainless steel.

The abovementioned mechanism should be able to exert an axial force of at least 10 Newtons, preferably at least 15 Newtons on the syringe piston assembly since the largest syringe conventionally used (30ml) generally applies a backward force of around 15 Newtons. It has been found that the feed mechanism of the present invention can exert an axial force of 60 Newtons and accordingly, the mechanism is suitable for dispensing fluid from all standard sizes of syringe.

The feed rate and diameter of the shaft can be used to provide a guideline for the required angle of inclination of the bearings with respect to the shaft using the following formula:

$$\theta = \text{Tan}^{-1} \text{ (Feed Rate in mm per revolution of shaft/(Diameter of the shaft in mm}^*\pi))$$

For example, for a feed rate of 0.5mm per revolution of the shaft which is in the order of 4.0mm in diameter:

$$\theta = \text{Tan}^{-1} (0.5/(4.00^*\pi)) = 2.2785 \text{ degrees, say } 2.3 \text{ deg.}$$

The present invention also provides for the return of the carriage and actuator to their original positions to enable a new syringe to be inserted into the assembly. This is achieved by disengagement of the middle bearing 27 from the shaft by means of a push spring mechanism 40 (see Figures 2 to 5) which enables the bearings to slide along the shaft, thereby allowing the carriage and actuator to be repositioned as desired.

Alternatively, the assembly may be provided with means for the automatic reversal of the carriage and actuator without changing the direction of rotation of the shaft. This may be achieved by providing means whereby the angle of inclination of the bearings relative to the shaft is reversed, i.e. the positioning of the bearings with respect to the shaft is altered to its corresponding mirror image.

The syringe driver assembly of the present invention is preferably provided with a mechanism for detection of an overload of the system, for example if an occlusion occurs in the line with results in a back pressure build up in the syringe, whereby detection of an overload causes the motor to stop.

In an alternative embodiment of the present invention, the three inclined bearings may be symmetrically spaced in one plane perpendicular to the shaft axis with the outer races of the bearings making radial contact with the shaft (not shown). In this configuration, the bearings may be spring loaded onto the shaft to control the contact force, removing the need for precision manufacture of the bearings to shaft location.

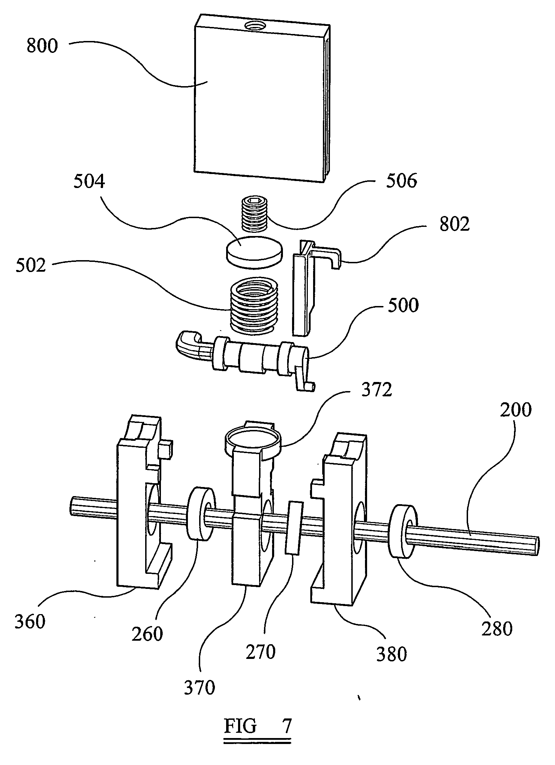

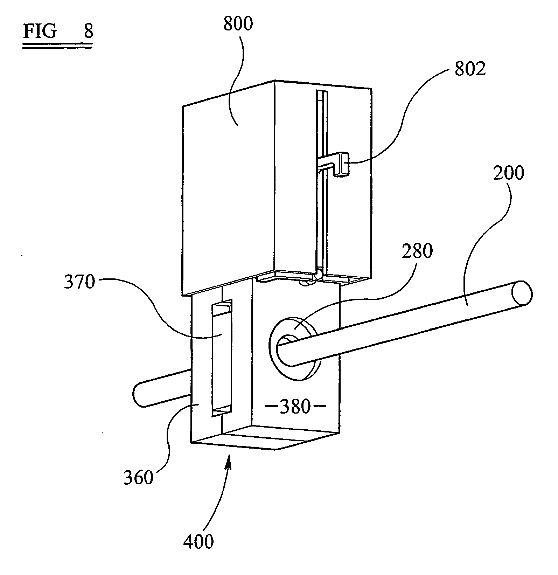

Figures 7 and 8 of the accompanying drawings illustrate a further embodiment of the present invention. For the sake of simplicity, only the parts of the mechanism that are involved with the movement of the bearings relative to the shaft are shown. Three oversized bearings 260, 270, 280 are mounted on a smooth shaft 200, the outer bearings 260, 280 each contacting the bottom of the shaft and the middle bearing 270 contacting the top of the shaft. Each bearing is twisted such that it contacts the shaft at two points along the breadth of the bearing. The middle bearing contacts the shaft at an opposite angle to the outer bearings.

Each bearing is mounted within its own casing 360, 370, 380 which are adapted to be fitted together to form a carriage 400 (see Figure 8). The casing 370 of the middle bearing is provided with a flange 372 for co-operating with a rotary cam 500 which has a spring 502 acting against it. A compression plate 504 abuts the top of the spring and a socket set screw flat point 506 is provided to contact the compression plate. The springs and compression plate are provided within a housing 800 which is also provided with a slideable clamp hook 802 for receiving a thumbplate of a syringe driver. This hook may or may not be moveable my means of the cam 500.

In this manner, rotation of the motor-driven shaft 200 causes movement of the bearings along the shaft. This imparts movement to the carriage 400 which is linked to the housing 800 which contacts the thumbplate of a syringe plunger thereby moving a plunger through a syringe barrel. Rotation of the cam 500 by means of a lever moves the casing containing the middle bearing 270 to lift it away from the shaft to disengage the bearing and enable the carriage to be slid freely along the shaft. The amount of force applied to the middle bearing can be adjusted by means of the spring, compression plate and socket set to adjust the load on the shaft.

## Claims

1. A syringe driver assembly comprising driver means for imparting translational movement to a syringe plunger, **characterised in that**, the driver means comprising a motor driven unthreaded shaft (200), at least one bearing (260, 270, 280) mounted obliquely to the shaft and having at least one point of contact therewith, and an actuator (800) linked to the at least one bearing for contacting a thumbplate of the plunger wherein rotation of the shaft causes movement of the at least one bearing along the shaft to affect movement of the actuator.

2. A syringe driver assembly as claimed in claim 1 wherein a single bearing is provided and the shaft is supported at one or more points along its length by a rotary member.

3. A syringe driver assembly as claimed in claim 2 wherein the rotary member is provided on an opposite side of the shaft to the contact point of the bearing.

4. A syringe driver assembly as claimed in claim 1 wherein at least three bearings (260, 270, 280) are provided with alternate bearings (260, 280) being mounted at the same angle relative to the shaft (200) and adjacent bearings (260, 270) being mounted at an opposing angle relative to the shaft.

5. A syringe driver assembly as claimed in any one of claims 1 to 4 wherein each bearing has a bore through which the shaft passes, the bore being larger than the outer circumference of the shaft.

6. A syringe driver assembly as claimed in claim 5 wherein the bearing has a pointed inner profile.

7. A syringe driver assembly as claimed in claim 5 wherein the bearing has a flat inner profile with a chamfered inner race.

8. A syringe driver assembly as claimed in claim 7 wherein each bearing is angled with respect to the shaft such that it contacts the shaft at at least two points.

9. A syringe driver assembly as claimed in any one of claims 4 to 8 wherein three bearings are provided, the outer bearings contacting the bottom of the shaft and the central bearing contacting the top of the shaft or vice versa.

10. A syringe driver assembly as claimed in any one of the preceding claims wherein the angle of inclination of each bearing (260, 270, 280) relative to the shaft (200) is less than 45 degrees.

11. A syringe driver assembly as claimed in any one of claims 1 to 4 wherein the inclined bearings are symmetrically spaced in one plane perpendicular to the shaft axis, the outer races of the bearings making radial contact with the shaft.

12. A syringe driver assembly as claimed in claim 12 wherein the bearing is spring loaded.

13. A syringe driver assembly as claimed in any one of the preceding claims wherein the or each bearing (260, 270, 280) is housed within a carriage (400) that is moveable with respect to the shaft (200).

14. A syringe driver assembly as claimed in claim 12 wherein the carriage (400) is connected to the actuator (800).

15. A syringe driver assembly as claimed in claim 14 wherein the carriage is provided with guides.

16. A syringe driver assembly as claimed in any one of the preceding claims wherein the means is provided for manually disengaging the at least one bearing (270) to enable it to slide independently of the shaft (200).

17. A syringe driver assembly as claimed in claim 16 wherein a bearing is springloaded with respect to the shaft whereby operation of the spring mechanism disengages the bearing from the shaft.

18. A syringe driver assembly as claimed in claim 16 wherein manual disengagement is affected by movement of a housing (370) containing a bearing (270) in a direction transverse to the shaft to lift the bearing from the shaft.

19. A syringe driver assembly as claimed in claim 18 wherein a cam (500) and lever is used.

20. A syringe driver assembly as claimed in any one of claims 1 to 15 wherein automatic means is provided for reversing the direction of travel of the bearings and actuator along the shaft.

21. A syringe driver assembly as claimed in any one of the preceding claims wherein the bearing is provided with adjustable biasing means (502, 504, 506).

**EP 1 694 384 B1**

**Patentansprüche**

1. Spritzenantriebsbaugruppe, die Antriebsmittel umfasst, um einem Spritzendruckkolben eine Translationsbewegung zu verleihen, **dadurch gekennzeichnet, dass** die Antriebsmittel eine motorgetriebene gewindelose Welle (200), wenigstens ein Lager (260, 270, 280), das schräg an der Welle angebracht ist und wenigstens einen Berührungspunkt mit derselben hat, und einen Stellantrieb (800), der mit dem wenigstens einen Lager verknüpft ist, um eine Daumenplatte des Druckkolbens zu berühren, umfasst, wobei ein Drehen der Welle eine Bewegung des wenigstens einen Lagers längs der Welle verursacht, um auf eine Bewegung des Stellantriebs einzuwirken.

2. Spritzenantriebsbaugruppe nach Anspruch 1, wobei ein einziges Lager bereitgestellt wird und die Welle an einem oder mehreren Punkten längs ihrer Länge durch ein sich drehendes Element getragen wird.

3. Spritzenantriebsbaugruppe nach Anspruch 2, wobei das sich drehende Element auf einer dem Berührungspunkt des Lagers gegenüberliegenden Seite der Welle bereitgestellt wird.

4. Spritzenantriebsbaugruppe nach Anspruch 1, wobei wenigstens drei Lager (260, 270, 280) bereitgestellt werden, wobei abwechselnde Lager (260, 280) in dem gleichen Winkel im Verhältnis zu der Welle (200) angebracht sind und benachbarte Lager (260, 270) in einem entgegengesetzten Winkel im Verhältnis zu der Welle angebracht sind.

5. Spritzenantriebsbaugruppe nach einem der Ansprüche 1 bis 4, wobei jedes Lager eine Bohrung hat, durch welche die Welle hindurchgeht, wobei die Bohrung größer ist als der Außenumfang der Welle.

6. Spritzenantriebsbaugruppe nach Anspruch 5, wobei das Lager ein zugespitztes Innenprofil hat.

7. Spritzenantriebsbaugruppe nach Anspruch 5, wobei das Lager ein flaches Innenprofil mit einem abgeschrägten inneren Laufring hat.

8. Spritzenantriebsbaugruppe nach Anspruch 7, wobei jedes Lager derart in Bezug auf die Welle abgewinkelt ist, dass es die Welle an wenigstens zwei Punkten berührt.

9. Spritzenantriebsbaugruppe nach einem der Ansprüche 4 bis 8, wobei drei Lager bereitgestellt werden, wobei die äußeren Lager die Unterseite der Welle berühren und das mittlere Lager die Oberseite der Welle berührt oder umgekehrt.

10. Spritzenantriebsbaugruppe nach einem der vorhergehenden Ansprüche, wobei der Neigungswinkel jedes Lagers (260, 270, 280) im Verhältnis zu der Welle (200) geringer als 45 Grad ist.

11. Spritzenantriebsbaugruppe nach einem der Ansprüche 1 bis 4, wobei die geneigten Lager in einer Ebene, senkrecht zu der Wellenachse, symmetrisch mit Zwischenraum angeordnet sind, wobei die äußeren Laufringe der Lager eine radiale Berührung mit der Welle herstellen.

12. Spritzenantriebsbaugruppe nach Anspruch 11, wobei das Lager federgespannt ist.

13. Spritzenantriebsbaugruppe nach einem der vorhergehenden Ansprüche, wobei das oder jedes Lager (260, 270, 280) innerhalb eines Schlittens (400) untergebracht ist, der in Bezug auf die Welle (200) bewegt werden kann.

14. Spritzenantriebshaugruppe nach Anspruch 12, wobei der Schlitten (400) mit dem Stellantrieb (800) verbunden ist.

15. Spritzenantriebsbaugruppe nach Anspruch 14, wobei der Schlitten mit Führungen versehen ist.

16. Spritzenantriebsbaugruppe nach einem der vorhergehenden Ansprüche, wobei das Mittel bereitgestellt wird, um das wenigstens eine Lager (270) von Hand auszurücken, um zu ermöglichen, dass es unabhängig von der Welle (200) gleitet.

17. Spritzenantriebsbaugruppe nach Anspruch 16, wobei ein Lager in Bezug auf die Welle federgespannt ist, wodurch ein Betätigen des Federmechanismus das Lager von der Welle ausrückt.

18. Spritzenantriebsbaugruppe nach Anspruch 16, wobei das Ausrücken von Hand beeinflusst wird durch eine Bewegung eines Gehäuses (370), das ein Lager (270) enthält, in einer Richtung, quer zu der Welle, um das Lager von der Welle anzuheben.

19. Spritzenantriebsbaugruppe nach Anspruch 18, wobei ein Nocken (500) und ein Hebel verwendet werden.

20. Spritzenantriebsbaugruppe nach einem der Ansprüche 1 bis 15, wobei selbsttätige Mittel bereitgestellt werden, um die Bewegungsrichtung der Lager und des Stellantriebs längs der Welle umzukehren.

21. Spritzenantriebsbaugruppe nach einem der vorhergehenden Ansprüche, wobei das Lager mit einstellbaren Vorspannmitteln (502, 504, 506) versehen ist.

**Revendications**

1. Assemblage pousse-seringue, comprenant un moyen d'entraînement pour conférer un mouvement de translation à un piston de seringue, **caractérisé en ce que** le moyen d'entraînement comprend un arbre non fileté entraîné par un moteur (200), au moins un palier (260, 270, 280) monté de manière oblique sur l'arbre et comportant au moins un point de contact avec celui-ci, et un dispositif d'actionnement (800) relié audit au moins un palier pour contacter une plaque pour le pouce du piston, la rotation de l'arbre entraînant le déplacement du au moins un palier le long de l'arbre pour affecter le déplacement du dispositif d'actionnement.

2. Assemblage pousse-seringue selon la revendication 1, comprenant un seul palier, l'arbre étant supporté au niveau d'un ou de plusieurs points le long de sa longueur par un élément rotatif.

3. Assemblage pousse-seringue selon la revendication 2, dans lequel l'élément rotatif est agencé sur un côté de l'arbre opposé au point de contact du palier.

4. Assemblage pousse-seringue selon la revendication 1, comprenant au moins trois paliers (260, 270, 280), les paliers alternés (260, 280) étant montés à un angle identique par rapport à l'arbre (200) et les paliers adjacents (260, 270) étant montés à un angle opposé par rapport à l'arbre,

5. Assemblage pousse-seringue selon l'une quelconque des revendications 1 à 4, dans lequel chaque palier comporte un alésage à travers lequel passe l'arbre, l'alésage étant plus grand que la circonférence externe de l'arbre.

6. Assemblage d'entraînement de seringue selon la revendication 5, dans lequel le palier a un profil interne pointu.

7. Assemblage pousse-seringue selon la revendication 5, dans lequel le palier a un profil intérieur plat avec une bague inférieure chanfreinée.

8. Assemblage pousse-seringue selon la revendication 7, dans lequel chaque palier est incliné à un angle par rapport à l'arbre, de sorte à contacter l'arbre au niveau d'au moins deux points.

9. Assemblage pousse-seringue selon l'une quelconque des revendications 4 à 8, comportant trois paliers, les paliers externes contactant la partie inférieure de l'arbre et le palier central contactant la partie supérieure de l'arbre ou vice-versa.

10. Assemblage pousse-seringue selon l'une quelconque des revendications précédentes, dans lequel l'angle d'inclinaison de chaque palier (260, 270, 280) par rapport à l'arbre (200) est inférieur à 45 degrés.

11. Assemblage pousse-seringue selon l'une quelconque des revendications 1 à 4, dans lequel les paliers inclinés sont espacés de manière symétrique dans un plan perpendiculaire à l'axe de l'arbre, les bagues extérieures des paliers établissant un contact radial avec l'arbre.

12. Assemblage pousse-seringue selon la revendication 11, dans lequel le palier est chargé par ressort.

13. Assemblage pousse-seringue selon l'une quelconque des revendications précédentes, dans lequel le ou chaque palier (260, 270, 280) est logé dans un chariot (400) pouvant se déplacer par rapport à l'arbre (200).

**14.** Assemblage pousse-seringue selon la revendication 12, dans lequel le chariot (400) est connecté au dispositif d'actionnement (800).

**15.** Assemblage pousse-seringue selon la revendication 14, dans lequelle le chariot comporte des guides.

**16.** Assemblage pousse-seringue selon l'une quelconque des revendication précédentes, comprenant un moyen pour dégager manuellement le au moins un palier (270) pour permettre son glissement indépendant de l'arbre (200).

**17.** Assemblage pousse-seringue selon la revendication 16, dans lequel un palier est chargé par ressort par rapport à l'arbre, l'actionnement du mécanisme à ressort entraînant ainsi le dégagement du palier de l'arbre.

**18.** Assemblage pousse-seringue selon la revendication 16, dans lequel le dégagement manuel est affecté par le déplacement d'un boîtier (30) contenant un palier (270), dans une direction transversale à l'arbre, afin de soulever le palier de l'arbre.

**19.** Assemblage pousse-seringue selon la revendication 18, utilisant une came (500) et un levier.

**20.** Assemblage pousse-seringue selon l'une quelconque des revendications 1 à 15, comprenant un moyen automatique pour inverser la direction du déplacement des paliers et du dispositif d'actionnement le long de l'arbre.

**21.** Assemblage pousse-seringue selon l'une quelconque des revendications précédentes, dans lequel le palier comporte un moyen poussoir ajustable (502, 504, 506).

FIG 1

FIG 2

FIG  3

FIG  4

FIG 5

FIG 6

800

504

506

802

502

500

372

200

360

260

270

380

280

370

<u>FIG 7</u>

FIG 8

**REFERENCES CITED IN THE DESCRIPTION**

*This list of references cited by the applicant is for the reader's convenience only. It does not form part of the European patent document. Even though great care has been taken in compiling the references, errors or omissions cannot be excluded and the EPO disclaims all liability in this regard.*

**Patent documents cited in the description**

- US 5006112 A